(19) **Europäisches Patentamt**

**European Patent Office**

**Office européen des brevets**

(11) **EP 1 514 929 A1**

(12) **EUROPEAN PATENT APPLICATION**

(43) Date of publication:
**16.03.2005 Bulletin 2005/11**

(51) Int Cl.⁷: **C12N 15/11**, C07H 21/00,
A61K 31/711, A61P 35/00,
A61P 35/04

(21) Application number: **03020789.8**

(22) Date of filing: **12.09.2003**

| | |
|---|---|
| (84) Designated Contracting States:<br>**AT BE BG CH CY CZ DE DK EE ES FI FR GB GR HU IE IT LI LU MC NL PT RO SE SI SK TR**<br>Designated Extension States:<br>**AL LT LV MK** | • **Berger, Martin**<br>**69126 Heidelberg (DE)**<br>• **Bäuerle, Tobias**<br>**69115 Heidelberg (DE)**<br>• **Armbruster, Franz**<br>**64625 Bensheim (DE)** |
| (71) Applicant: **Deutsches Krebsforschungszentrum Stiftung des öffentlichen Rechts**<br>**69120 Heidelberg (DE)** | (74) Representative: **Schüssler, Andrea, Dr.**<br>**Kanzlei Huber & Schüssler**<br>**Truderinger Strasse 246**<br>**81825 München (DE)** |
| (72) Inventors:<br>• **Adwan, Hassan**<br>**69121 Heidelberg (DE)** | |

(54) **Antisense oligonucleotides for prevention of metastasis formation of cancer cells**

(57) Described are antisense oligonucleotides targeted to the gene encoding osteopontin, bone sialoprotein II and/or osteonectin, wherein said oligonucleotides inhibit the expression of said gene(s). Moreover, the therapeutic use of said antisense oligonucleotides is described, e.g., for the treatment or prevention of cancer/ metastasis, preferably osteolytic metastasis or metastasis of breast cancer cells.

**EP 1 514 929 A1**

**Description**

The present invention relates to antisense oligonucleotides targeted to the gene encoding osteopontin (OPN), bone sialoprotein II (BSP II) and/or osteonectin (ON), wherein said oligonucleotides inhibit the expression of said gene (s). The present invention also relates to the therapeutic use of said antisense oligonucleotides, e.g., for the treatment or prevention of bone cancer or metastasis of several types of cancer, preferably osteolytic metastasis or metastasis of breast cancer cells.

Cancer is a life threatening disease not as a result of the primary tumor which can be removed surgically in the vast majority of cases but from its metastatic spread to adjacent and distal sites in the body. Currently used diagnostic techniques do not accurately reflect progression or metastatic spread of a cancer, but recently some evidence has accumulated with respect to markers which are related to the development of progression and metastasis. One molecule of this promising group of markers is the secreted adhesive glycophosphoprotein OPN. This molecule has recently been recognized as a lead marker of human colon cancer progression as shown by a significant correlation between osteopontin protein expression and advanced tumor stage [1]. Similarly, OPN was found to be a metastasis associated protein in human breast cancer in that over-expression of OPN in breast tumors as well as in blood of patients was highly correlated with tumor progression [2, 3]. OPN was initially described as a protein the secretion of which was elevated in many transformed cells in culture. Besides this fact it was also identified as a major noncollagenous protein in bone. Moreover, it is a calcium-binding protein that is important in bacterial resistance and immune activity as well as in cell adhesion, signaling, migration and survival of various cells, in addition to being a potent regulator of osseous and ectopic calcification. OPN and bone sialoprotein BSP II are members of the SIBLING (small integrin-binding ligand, N-linked glycoprotein) family of genetically related proteins that are clustered on the long arm of human chromosome 4 [4]. These proteins often function by bridging two proteins of fixed structures into a biologically active complex. The structural similarity of BSP II with OPN is paralleled by the observation that both proteins are expressed by certain tumor cells such as breast cancer [5, 6], as well as colon, prostate [7] and lung cancer [8], and have been related to the pathogenesis of bone metastasis [9]. The calcium binding properties of BSP II and its role in initiating and controlling hydroxylapatite crystallization have been related to the formation of micro-calcification in breast cancer. Osteonectin (ON) is a third molecule that has been related to progression of human colorectal [10] and urinary bladder cancer [11] as well as to bone metastasis [12]. It is a non-collagenous, calcium binding glycoprotein which has been mapped to the long arm of chromosome 5 and is related to wound healing and angiogenesis [21]. The current situation is that a successful and specific treatment of cancers/metastasis cannot be achieved.

Therefore, it is the object of the present invention to provide a therapeutic means for the specific treatment/ prevention of cancers/metastasis.

According to the invention this is achieved by the subject matters defined in the claims. For a specific treatment, antisense oligonucleotides were identified that are capable of reducing the expression levels of OPN, BSP II and/or ON. The activity of these ASOs was determined by Western Blot and by inhibition of colony formation as well as of metastasis formation of pre-exposed MDA-MB-231 human mammary carcinoma cells. This cell line was chosen for its ability to express the three proteins, to form colonies, and to induce lytic bone metastasis in nude rats. The antisense oligonucleotides were effective in reducing protein levels of OPN, BSP II and ON. In human MDA-MB-231 breast cancer cells, the maximum inhibition of protein expression ranged from 84% (OPN) to 75% (BSP II) and 70% (ON). Erucylphospho-NNN-trimethylpropanolamine (ErPC$_3$) was used as positive control and combination partner. Exposure to ErPC$_3$ inhibited colony formation of MDA-MB-231 cells by 11% (10µM), 45% (14µM) and 78% (20µM). The clonogenicity of breast cancer cells was reduced by 15%, 11%, 8% (5µM), 39%, 19%, 14% (10µM) and 46%, 39%, 21% (20µM) in response to ASO-OPN-04, ASO-BSPII-06 and ASO-ON-03, respectively. Combination of ErPC$_3$ with the ASOs caused additive combination effects. Pre-exposure to the ASOs, but not to the NSO, inhibited formation of osteolytic metastasis in 3 of 4 (ASO-OPN-04, p<0,03) and 2 of 4 (ASO-BSPII-06) nude rats, and reduced metastasis lesions significantly (T/C% = 4.3 and 9.1, p=0.05, respectively). Thus, it can be concluded that down-regulation of OPN, BSPII and/or ON reduces colony formation of MDA-MB-231 cells and formation of osteolytic metastasis in nude rats. Accordingly, the antisense oligonucleotides of the invention are expected to be useful for therapy or prevention of cancer/metastasis.

**Legends to figures**

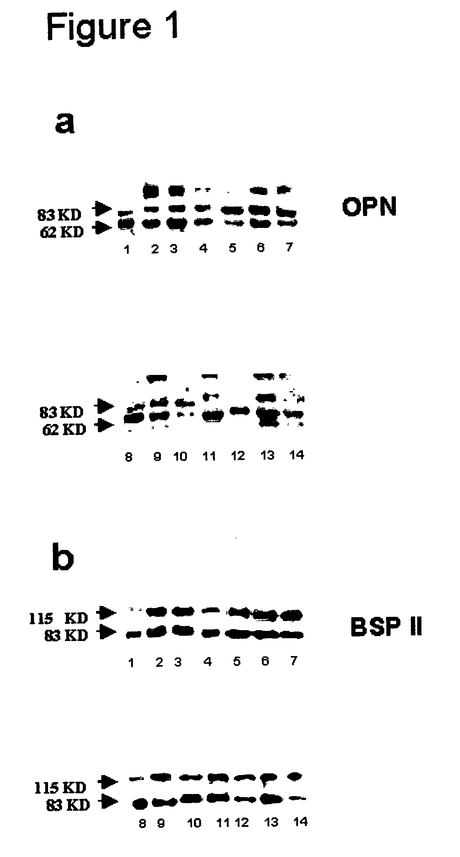

Figure 1: Detection of osteopontin (a) and bone sialoprotein II (b) in various cell lines

(a) 1 = urine, 2 = MDA MB-231, 3 = MDA MB-435s, 4 = MDA MB-231F, 5 = MCF-7
6 = T47-D, 7 = SAOS, 8 = urine, 9 = HT-29, 10 = BV-173, 11 = HL-60, 12 = K-562
13 = ACHN, 14 = medium.

The mouse monoclonal antibody against human osteopontin recognizes multiple bands with an apparent size ranging from 60 to 100 kD.

(b) 1 = BSP - standard, 2 = MDA MB-231, 3 = MDA MB-435, 4 = MDA MB-231 F,

5 = MCF-7, 6 = T47-D, 7 = SAOS, 8 = BSP - standard, 9 = HT-29, 10 = BV-173,

11 = HL-60, 12 = K-562, 13 = ACHN, 14 = medium.

The rabbit polyclonal antibody against human bone sialoprotein II recognizes two bands with an apparent size ranging from 80 to 160 kD. Erythrocytes served as negative control for both, the OPN and BSP II antibodies and, in addition, large bowel mucosa and white blood cells served as negative control for BSP II. Urine was taken from a normal subject and the medium was from MDA-MB-231 cells growing without FBS.

Figure 2: Effect of treating MD-MB-231 cells with various antisense oligonucleotides (10µM) directed against RNA from osteopontin (top), bone-sialoprotein II (middle), and osteonectin (bottom) or a nonsense oligonucleotide (10µM)

The expression following repeated treatment **(a, b)** and single treatment **(c)** is given in percent relative to control treated with lipofectamine. The expression of actin (lower part of **a, b, and c**) is given for comparison.

Figure 3 : X-ray scans of three rats' hind legs

showing **(a)** no osteolysis after injection of MDA-MB-231 cells pre-exposed to ASO-OPN-04, **(b)** a minute osteolytic lesion in the tibia only after injection of MDA-MB-231 cells preexposed to ASO-BSPII-6 and (c) multiple lesions in the femur, tibia and fibula after injection of control MDA-MB-231 cells.

**Abbreviations used**

OPN, osteopontin; BSP II, bone sialoprotein II; ON, osteonectin; ErPC$_3$, Erucylphospho-NNN-trimethylpropanolamine; ASO, antisense oligonucleotide; NSO, nonsense oligonucleotide; SIBLING, small integrin-binding ligand, N-linked glycoprotein; ECM, extracellular matrix; FBS, fetal bovine serum; HUSAR, Heidelberg UNIX sequence Analysis Resource.

Accordingly, the present invention relates to a pharmaceutical composition containing at least one compound 8 to 50 nucleobases in length targeted to (a) nucleic acid molecule(s), preferably mRNA, encoding osteopontin, bone sialoprotein II and/or osteonectin, wherein said compound(s) specifically hybridize(s) with and inhibit(s) the expression of the nucleic acid(s) encoding osteopontin, bone sialoprotein II and/or osteonectin.

"Targeting" an antisense compound to a particular nucleic acid, in the context of this invention, is a multistep process. The targeting process also includes determination of a site or sites within the gene for the antisense interaction to occur such that the desired effect, i.e., inhibiton of expression of the protein, will result. Within the context of the present invention, a preferred intragenic site is (a) the region encompassing the translation initiation or termination codon of the open reading frame (ORF) of the gene or (b) a region of the mRNA which is a "loop" or "bulge", i.e., not part of a secondary structure. Once one or more target sites have been identified, oligonucleotides are chosen which are sufficiently complementary to the target, i.e., hybridize sufficiently well and with sufficient specificity, to give the desired 30 effect. In the context of this invention, "hybridization" means hydrogen bonding, which may be Watson-Crick, Hoogsteen or reversed Hoogsteen hydrogen bonding, between complementary nucleoside or nucleotide bases. "Complementary," as used herein, refers to the capacity for precise pairing between two nucleotides. For example, if a nucleotide at a certain position of an oligonucleotide is capable of hydrogen bonding with a nucleotide at the same position of a DNA or RNA molecule, then the oligonucleotide and the DNA or RNA are considered to be complementary to each other at that position. The oligonucleotide and the DNA or RNA are complementary to each other when a sufficient number of corresponding positions in each molecule are occupied by nucleotides which can hydrogen bond with each other. Thus, "specifically hybridizable" and "complementary" are terms which are used to indicate a sufficient degree of complementarity or precise pairing such that stable and specific binding occurs between the oligonucleotide and the DNA or RNA target. It is understood in the art that the sequence of an antisense compound need not be 100% complementary to that of its target nucleic acid to be specifically hybridizable. An antisense compound is specifically hybridizable when binding of the compound to the target DNA or RNA molecule interferes with the normal function of the target DNA or RNA to cause a loss of utility, and there is a sufficient degree of complementarity to avoid nonspecific binding of the antisense compound to non-target sequences under conditions in which specific binding is desired, i.e., in the case of therapeutic treatment.

The skilled person can generate antisense compounds according to the present invention on the basis of the known DNA sequences for OPN, BSP II and ON; see Example 1(C), below. "Oligonucleotide" refers to an oligomer or polymer of ribonucleic acid (RNA) or deoxyribonucleic acid (DNA) or mimetics thereof. This term includes oligonucleotides composed of naturally-occurring nucleobases, sugars and covalent internucleoside (backbone) linkages as well as oligonucleotides having non-naturally-occurring portions which function similarly. Such modified or substituted oligonucleotides are often preferred over native forms because of desirable properties such as, for example, enhanced cellular uptake, enhanced affinity for nucleic acid target and increased stability in the presence of nucleases. While antisense oligonucleotides are a preferred form of the antisense compound, the present invention comprehends other oligomeric antisense compounds, including but not limited to oligonucleotide mimetics such as are described below. The antisense compounds in accordance with this invention comprise from about 8 to about 50 nucleobases (i.e. from about 8 to about 50 linked nucleosides). Particularly preferred antisense compounds are antisense oligonucleotides, even more preferably those comprising from about 15 to about 25 nucleobases. Antisense compounds include ribozymes, external guide sequences (EGS), oligonucleotides (oligozymes), and other short catalytic RNAs and RNAi (siRNA) or catalytic oligonucleotides which hybridize to the target nucleic acid and inhibit its expression.

Alternatively, the pharmaceutical composition of the invention contains a vector allowing to transcribe an antisense oligonucleotide of the invention, e.g., in a mammalian host. Preferably, such a vector is a vector useful for gene therapy. Preferred vectors useful for gene therapy are viral vectors, e.g. adenovirus, herpes virus, vaccinia, or, more preferably, an RNA virus such as a retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotide encoding a sugar, a glycolipid, or a protein, preferably an antibody. Those skilled in the art know additional methods for generating target specific vectors. Further suitable vectors and methods for in vitro- or in vivo-gene therapy are described in the literature and are known to the persons skilled in the art; see, e.g., WO 94/29469 or WO 97/00957.

In order to achieve expression only in the target organ, e.g., a tumor to be treated, the DNA sequences for transcription of the antisense oligonucleotides can be linked to a tissue specific promoter and used for gene therapy. Such promoters are well known to those skilled in the art (see e.g. Zimmermann et al., (1994) Neuron 12, 11-24; Vidal et al.; (1990) EMBO J. 9, 833-840; Mayford et al., (1995), Cell 81, 891-904; Pinkert et al., (1987) Genes & Dev. 1, 268-76).

Preferably, an antisense compound of the pharmaceutical composition of the present invention has the nucleic acid sequence (a) 5'-CTA ACT TAA AAA ACA AAA GA-3' (ASO-OPN-04), 5'-GCT-TTC-TTC-GTT-TTC-ATT-TC-3' (ASO-BSP II-06), 5'-GGG GGC TGG GCA GCT GGT GG-3' (ASO-ON-03) or 5'-ATG TTA TAG TTC TTC TCG AA-3' (ASO-ON-05) or (b) a nucleic acid sequence which has at least 70%, preferably at least 75%, 80%, 85% or 90%, identity with a nucleic acid sequence of (a).

Within an oligonucleotide structure, the phosphate groups are commonly referred to as forming the internucleoside backbone of the oligonucleotide. The normal linkage or backbone of RNA and DNA is a 3' to 5' phosphodiester linkage. Specific examples of preferred antisense compounds useful in the present invention include oligonucleotides containing modified backbones or non-natural internucleoside linkages. Oligonucleotides having modified backbones include those that retain a phosphorus atom in the backbone and those that do not have a phosphorus atom in the backbone. Modified oligonucleotide backbones which can result in increased stability are known to the person skilled in the art, preferably such modification is a phosphorothioate linkage.

A preferred oligonucleotide mimetic is an oligonucleotide mimetic that has been shown to have excellent hybridization properties, and is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone (see, e.g., Nielsen et al., Science, 25, **1991**, 254, 1497-1500.)

Modified oligonucleotides may also contain one or more substituted or modified sugar moieties. Preferred oligonucleotides comprise one of the following at the 2' position: OH; F; 0-, S-, or N-alkyl; 0-, S-, or N-alkenyl; 0-, S- or N-alkynyl; or O-alkyl-O-alkyl, wherein the alkyl, alkenyl and alkynyl may be substituted or unsubstituted $C_1$ to $C_{10}$ alkyl or $C_2$ to $C_{10}$ alkenyl and alkynyl. A particularly preferred modified sugar moiety is a 2'-O-methoxyethyl sugar moiety.

Oligonucleotides of the invention may also include nucleobase modifications or substitutions. Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine etc., with 5-methylcytosine substitutions being preferred since these modifications have been shown to increase nucleic acid duplex stability.

Another modification of the oligonucleotides of the invention involves chemically linking to the oligonucleotide one or more moieties or conjugates which enhance the activity, cellular distribution or cellular uptake of the oligonucleotide. Such moieties include lipid moieties such as a cholesterol moiety, cholic acid, a thioether, a thiocholesterol, an aliphatic chain, e.g., dodecandiol or undecyl residues, a phospholipid, a polyamine or a polyethylene glycol chain, or adamantane acetic acid, a palmityl moiety, or an octadecylamine or hexylamino-carbonyl-oxycholesterol moiety.

The present invention also includes antisense compounds which are chimeric compounds. "Chimeric" antisense compounds or "chimeras," in the context of this invention, are antisense compounds, particularly oligonucleotides, which contain two or more chemically distinct regions, each made up of at least one monomer unit, i.e., a nucleotide in the case of an oligonucleotide compound. These oligonucleotides typically contain at least one region wherein the oligonucleotide is modified so as to confer upon the oligonucleotide increased resistance to nuclease degradation, increased cellular uptake, and/or increased binding affinity for the target nucleic acid. An additional region of the oligonucleotide may serve as a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target, thereby greatly enhancing the efficiency of oligonucleotide inhibition of gene expression. Consequently, comparable results can often be obtained with shorter oligonucleotides when chimeric oligonucleotides are used, compared to phosphorothioate deoxyoligonucleotides hybridizing to the same target region. Chimeric antisense compounds of the invention may be formed as composite structures of two or more oligonucleotides, modified oligonucleotides, oligonucleosides and/or oligonucleotide mimetics as described above. Such compounds have also been referred to in the art as hybrids or gapmers.

The antisense compounds of the invention may be conveniently and routinely made through the well-known technique of solid phase synthesis. Equipment for such synthesis is sold by several vendors. Any other means for such synthesis known in the art may additionally or alternatively be employed. Similar techniques can be used for preparing oligonucleotides such as the phosphorothioates and alkylated derivatives.

In a further preferred embodiment, the pharmaceutical composition of the present invention additionally contains a cytostatic alkylphosphocholine. Suitable alkylphosphocholines are known to the skilled person. A preferred example of a cytostatic alkylphosphocholine is erucylphospho-NNN-trimethylpropanolamine ($ErPC_3$).

Examples of suitable pharmaceutical carriers for the pharmaceutical composition of the present invention are well known in the art and include phosphate buffered saline solutions, water, emulsions, such as oil/water emulsions, various types of wetting agents, sterile solutions etc. Such carriers can be formulated by conventional methods and can be administered to the subject at a suitable dose. Administration of the suitable compositions may be effected by different ways, e.g. by intravenous, intraperetoneal, subcutaneous, intramuscular, topical or intradermal administration. The route of administration, of course, depends on the nature of the disease and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind and stage of the disease, e.g., tumor, general health and other drugs being administered concurrently.

The delivery of the antisense compounds of the present invention can be achieved by direct application or, preferably, by using a recombinant expression vector such as a chimeric virus containing these compounds or a colloidal dispersion system. Direct application to the target site can be performed, e.g., by ballistic delivery, as a colloidal dispersion system or by catheter to a site in artery. The colloidal dispersion systems which can be used for delivery of the above antisense compounds include macromolecule complexes, nanocapsules, microspheres, beads and lipid-based systems including oil-in-water emulsions (mixed), micelles, liposomes and lipoplexes, The preferred colloidal system is a liposome. Some of these systems (in particular liposomes) provide a slow and/or continuous release of the active compound (antisense compound) which is preferably in some case. Organ-specific or cell-specific liposomes can be used in order to achieve delivery only to the desired tissue. The targeting of liposomes can be carried out by the person skilled in the art by applying commonly known methods. This targeting includes passive targeting (utilizing the natural tendency of the liposomes to distribute to cells of the RES in organs which contain sinusoidal capillaries) or active targeting (for example by coupling the liposome to a specific ligand, e.g., an antibody, a receptor, sugar, glycolipid, protein etc., by well known methods). In the present invention monoclonal antibodies are preferably used to target liposomes to specific tissues, e.g. tumor tissue, via specific cell-surface ligands.

The present invention also relates to the use of the compounds of the invention for the preparation of a pharmaceutical composition for the treatment or prevention of bone cancer (primary tumor) or metastasis of several cancer types, preferably for treatment/prevention of metastasis of cancer of the breast, colon, prostate, bone, urinary bladder or colorectal cancer.

The following examples illustrate the invention.

**Example 1**

**General Methods**

(A) Cells

Ten cell lines were used for detecting OPN, BSP II, and ON. They were selected from various organs to establish a possibly differential expression of the three proteins. Therefore, the panel consisted of cancer cells derived from the skeleton (SAOS-2), as well as of cells metastasizing into the skeleton (MDA-MB-231 and MDA-MB-435S). In addition, cells without apparent affinity to the skeleton were used for comparison (K562, HL-60, BV-173, MCF-7, T47D, HT29, and ACHN). The cell lines were obtained from the ATCC or DSZM, respectively, and were grown in media and supplements as recommended by the two organizations (fetal bovine serum, L-glutamine, insulin, Na-HCO$_3$, glucose, hepes, and pyruvate, all from Sigma, Taufkirchen, Germany). For determining the potential secretion of osteopontin and bone sialoprotein, a MDA-MB-231 subline was generated, which was selected by continuously reducing the FBS content of the medium, until the cells kept growing without FBS. This cell line was denoted as MDA-MB-231$^F$. All cells were kept in log-phase, and passaged 1-3 times per week depending of their growth rate, and maintained under standard conditions (37°C, humidified atmosphere, 5% CO$_2$).

(B) Clonogenicity assay

For determining the response of MDA-MB-231 colony formation to exposure with antisense oligonucleotides and erucylphospho-NNN-trimethylpropanolamine (ErPC$_3$) the following procedure was used. MDA-MB-231 cells were treated for various periods of time (see below), harvested and counted. Cells (5000) were then transferred into semi-solid medium containing 0.8% RPMI-methylcellulose and 30% FBS. Cells were plated onto 3.5 cm Petri-dishes (1ml/dish) (NUNC, Wiesbaden, Germany) and usual cell culture conditions (37°C, 5% CO$_2$ in humidified air) were applied. Colony formation (clusters of 30 or more cells) was visualized by staining with 3-[4,5-dimethylthiazol-2-yl]-2,5-diphenyltetrazolium-bromide (MTT; Serva, Heidelberg, Germany) and the resulting dark blue colonies were scored by an inverted microscope after 5-7 days. At least three dishes per treatment protocol were used.

(C) Selection of antisense oligonucleotides

The suitability of ten different antisense oligonucleotides (ASOs) per gene was predicted by using the HUSAR program 'Mfold' [13,14,15], which takes RNA folding into account. Application of this program onto the cDNA sequence of osteopontin (acc. no. gi:3360431), bone sialoprotein II (acc. no. gi:11435526) and osteonectin (acc. no. gi:14124969) resulted in the recognition of RNA stretches that probably contain bulges or loops, i.e. that are preferentially single stranded and thus allow access of DNA antisense structures. ASOs of 20 base pair lengths were selected against these single stranded regions and synthesized with a phosphorothioate backbone, to increase stability against degrading enzymes (Table 1).

## Table 1
### Antisense oligonucleotides selected against osteopontin, bone sialoprotein, and osteonectin by using the HUSAR MFold program (Dr. Zuker).

| No. | osteopontin cDNA (OPN)[1] | bone sialoprotein cDNA (BSPII)[1] | osteonectin cDNA (ON)[1] |
|---|---|---|---|
| ASO-01 | bp. 73 - 92 5'-CTC ATG GTA GTG AGT TTT CC-3' | bp. 46 - 65 5'-TGA TTG CTT CCT CTG GCA GT-3' | bp. 5 - 24 5'- AGA TCC AGG CCC TCA TGG TG -3' |
| ASO-02 | bp. 661 - 680 5'-TTC AGG TCC TGG GCA ACG GG-3' | bp. 53 - 73 5'-ATT TTG GTG ATT GCT TCC TC -3' | bp. 162 - 181 5'- CCA TCA TCA AAT TCT CCT AC -3' |
| ASO-03 | bp. 662 - 681 5'-GTT CAG GTC CTG GGC AAC GG-3 | bp. 328 - 347 5'-CTT CAT TGT TTT CTC CTT CA-3' | bp. 301 - 320 5'- GGG GGC TGG GCA GCT GGT GG -3' |
| ASO-04 | bp. 1343-1362 5'-CTA ACT TAA AAA ACA AAA GA -3' | bp. 331 - 350 5'-ATT CTT CAT TGT TTT CTC CT-3 | bp. 489 - 508 5'- ATG CGC AGG GGG AAT TCG GT |
| ASO-05 | bp. 1346 - 1365 5'-ACA CTA ACT TAA AAA ACA AA -3' | bp. 505 - 524 5'-CTT CAT CAC TTT CCT TCT CT-3' | bp. 666 - 685 5'- ATG TTA TAG TTC TTC TCG AA -3' |
| ASO-06 | bp193 - 212 5'-GTG GCC ACA GCA TCT GGG TA -3' | bp. 546 - 565 5'-GCT TTC TTC GTT TTC ATT TC-3' | bp. 813 - 832 5'- AGG TCA CAG GTC TCG AAA AA -3' |
| ASO-07 | bp193 - 212 5'-GTG GCC ACA GCA TCT GGG TA-3' | bp. 706 - 725 5'-TTC CGG TCT CTG TGG TGT CT-3' | bp. 830 - 849 5'- TGT ACT TGT CAT TGT CCA GG -3' |
| ASO-08 | 195 - 214 5'-ATG TGG CCA CAG CAT CTG GG-3' | bp. 711 - 730 5'-CTG CCT TCC GGT CTC TGT GG -3' | bp. 928 - 947 5'- GAA TCC GGT ACT GTG GAA GG -3' |
| ASO-09 | 556 - 575 5'-GGT CTG CGA AAC TTC TTA GA-3' | 1003 - 1022 5'-ACT GGT GGT GGT AGT AAT TC-3' | bp. 976 - 995 5'- CAA ACA TTT TAA ACA TTG GG -3' |
| ASO-10 | 559 - 578 5'-TCA GGT CTG CGA AAC TTC TT-3' | bp. 1005 - 1024 5'-TCA CTG GTG GTG GTA GTA AT-3' | bp. 1882 - 1901 5'- TTG AAG TTT CTC TTG ATT AA -3' |

The following sequence from HBV was used as a nonsense control:
5'-GCG AGG GAG TTC TTC TTC TA-3'

[1] The combination of the abbreviation of the gene and the number of a given antisense oligonucleotide (ASO) was used for its naming.

Controls included a nonsense oligonucleotide (NSO) derived from HBV genome which served as backbone analog to control for unspecific effects of a 20 bp long phosphorothioate oligomer. In addition its base composition differed only slightly from the mean composition of all ASOs used (A: 3 vs. 4.1; C: 4 vs. 4.4; G: 6 vs. 4.8; T: 7 vs. 6.6). Sequence specific effects were controlled by using ASOs differing by three base pairs with regard to the respective cDNA sequence. Finally, the use of ten ASOs per target gene allowed to include sequences that were scrambled (identical in base composition) except for two bases that were permutated.

(D) Exposure of cells to ASOs and ErPC$_3$

Stock solutions of ASOs in distilled water were diluted to appropriate concentrations with phosphate buffered saline. For transfecting cells with ASOs, lipofectamine (Lipofectin, Invitrogen, Karlsruhe, Germany) or electroporation was used.

(E) Electroporation

Pending on the duration of treatment, $5\times10^5$ - $3\times10^6$ cells were suspended in one ml RPMI 1640 medium without phenol red containing 10 μM concentrations of the respective ASO or NSO. For transfection, the cells were pulsed two or three times for 2ms at 450 or 350V, respectively, in a 800 μl cuvette [Eppendorf, Hamburg, Germany] using an electroporation impulse generator (EPI2500, Dr. L. Fischer, Heidelberg, Germany) with the capacitance set at 1200 μF. Thereafter the cells were plated onto 6 wells plates and grown for 48 - 72 h.

(F) Lipofectamine

Pending on the duration of treatment, $1\times10^5$ - $3\times10^5$ cells were transferred onto 6 well plates (Nunc, Wiesbaden, Germany) and grown for 24h. For transfection, ASO stock solutions were prepared in medium without FBS. According to the protocol of the manufacturer, the respective ASO stock solution was mixed with lipofectamine to allow ASO-liposome complexes to form. To 200 μl medium containing ASO quantities resulting in 10 μM (Western blot and in vivo model) and in 5, 10, or 20 μM (clonogenicity assay) final ASO-concentrations, an equal volume of medium without FBS was added containing 5, 10, or 20 μl lipofectamine, respectively. In addition to the ASO treatment, a NSO control (20 μM final concentration with 20 μl lipofectamine) and a lipofectamine control (20 μl) were used. Based on the equilibrium of the ASO-liposome complex formation, the relatively low amount of lipofectamine used resulted in effective ASO concentrations that were at least by a factor of ten lower than those in the medium used for transfection. After 12h the medium was changed, and cells were further grown for 48 - 72h. In the case of a sequential combination treatment this first cycle was repeated with the respective combination partner.

(G) Erucylphospho-NNN-trimethylpropanolamine

The alkylphosphocholine ErPC$_3$ was synthesized and provided by Prof. H. Eibl (MPI of Biophysical Chemistry, Göttingen, FRG). For treating MDA-MB-231 cells, ErPC$_3$ which was kept as stock solution in ethanol and PBS (10 mM; ratio of diluents 1:1; V:V), was diluted in PBS resulting in final concentrations of 10, 14, and 20 μM. The medium containing ErPC3 was changed after 24h in all experiments and the cells were further grown for 48 - 72h without ErPC$_3$ (for details of sequential exposure see below).

(H) Combination Treatment

Sequential combination treatment consisted of two or three treatment cycles. The two cycle treatment started either with the ASO and was followed by ErPC$_3$ or was performed with the reverse sequence. The three cycle treatment started either with two cycles of ASO followed by ErPC$_3$ or an initial exposure to ErPC$_3$ was followed by two cycles with an ASO.

(I) Statistics and evaluation of combination effects

Colony counts are presented as mean with corresponding standard deviation. For comparison of incidences between treatment groups the $\chi^2$ test was used and for comparison of the lesion sizes the Kruskal-Wallis test was applied [16]. P values $\leq 0.05$ were considered significant. Predicted theoretical values of combinations were calculated according to the equation: **C = a$\times$b / 100,** where a and b are colony counts in percent of untreated control obtained in response to single agents. For each concentration applied theoretical values were calculated and compared with the real value of the combination. In addition, isobologram and multiple drug effect analysis was examined by a suitable program (CalcuSyn, Biosoft, Ferguson, USA) to study the nature of the interaction observed [17,18]. For this purpose, the dose-effect curve for each drug alone was determined based on the experimental observations using the median-effect principle; the combination index (CI) for each combination was then calculated according to the following equation:

$$CI = (D)_1 / (D_x)_1 + (D)_2 / (D_x)_2 + (D1) \times (D_2) / (D_x)_1 \times (D_x)_2$$

where $(D)_1$ and $(D)_2$ are the doses of drug 1 and drug 2 that have x effect when used in combination and $(D_x)_1$ and $(D_x)_2$ are the doses of drug 1 and drug 2 that have the same x effect when used alone. When CI = 1, this equation represents the conservation isobologram and indicates additive effects. CI values less than 1.0 indicate a more than expected additive effect (synergism).

(J) Western blotting

Transfected cells were incubated, harvested and washed in PBS. Thereafter cells were counted (Neubauer chamber) and cell pellets ($2 \times 10^6$ cells) were suspended in 200 μl buffer [0.1 M NaCl, 0.01 M Tris. Cl (pH 7.6), 0.001 M EDTA (pH 8.0)] containing 1 mM PMSF (Sigma), 50 μg/ml aprotinin (Sigma), 1mM $Na_3 VO_4$ , and 2 mg/ml leupeptin (Sigma). After that the cells were lysed by adding 200 μl of lysis buffer [100 mM Tris. Cl (pH 6.8), 200 mM dithiothreitol (Serva, Heidelberg, Germany), 4% SDS, 0.2% bromophenol blue and 20% glycerol]. After vigorously vortexing, lysates were boiled for 10 minutes in a water bath at 100°C and centrifuged at 14000 rpm for 10 min. at room temperature. The protein concentration of the lysate was determined using the BCA protein assay from Pierce (Rockford, Illinois, USA) according to the manufacturer's recommendation. Cell lysates corresponding to 150,000 cells were loaded onto a 6% SDS polyacrylamide gel, separated by electrophoresis and transferred to a PVDF membrane, blocked with 5% skimmed milk in PBS and incubated with the respective first antibody (OPN: rabbit-antihuman polyclonal or mouse - antihuman monoclonal antibody; ON: rabbit-antihuman polyclonal; BSP II chicken-antihuman or rabbit-antihuman polyclonal [all from Immundiagnostik, Bensheim, Germany]) for 2h in PBS; containing 0.5% milk and 0.5% Triton X-100. After washing with PBS, containing 0.05% Tween 20, an HRP-conjugated anti-mouse, anti-rabbit or anti-chicken secondary antibody (all from Immundiagnostik, Bensheim, Germany) and ECL (Amersham Biosciences, Freiburg, Germany) were used to detect the respective proteins by exposing the membrane to an X ray film (Kodak Biomax, Rochester, New York). To control for variations in loading, the membranes were stripped for 30 min. at 56°C in stripping solution (62.5 mM Tris HCl, (pH 6.8); 2% SDS; 0.007% β-mercaptoethanol). Thereafter they were reprobed with an antibody against β-actin (mouse monoclonal antibody, Santa Cruz Biotechnology, Santa Cruz, CA, USA), and a secondary goat anti mouse polyclonal antibody (Santa Cruz Biotechnology).

(K) Evaluation of band intensity for Western blot research

The X-ray films were processed by an automatic developing machine (Curix 60, Agfa, Köln, Germany) and the resulting images were scanned by using a digital imaging Program (Adobe photoshop 6.0, Adobe Systems Incorporated). Digitized bands of the respective proteins were given as percent of control and corrected for differences in loading by referring to the intensity of the β-actin band.

(L) Animals and husbandry

Nude rats (RNU strain) were obtained from Harlan (Harlan comp, Borchen, Germany) at an age of 6 to 8 weeks. They were housed under specific pathogen free conditions in a mini-barrier system of the central animal facility. Autoclaved feed and water was given ad *libitum* to the animals which were maintained under controlled conditions (21 ± 2°C room temperature, 60% humidity, 12 h light dark rhythm).

(M) In-vivo model

In order to induce loco-regional bone metastasis, and to investigate a preventive effect of the ASOs, $10^5$ MDA-MB-231 cells (control or pre-exposed to ASOs) were injected into a secondary muscular branch of the femoral artery of a nude rat. After an average of 28 days following the injection of control cells, lytic metastases could be detected by X-rays. The size of these lesions, which exclusively occurred in the femur, tibia and fibula of the animals, was recorded for up to 10 weeks.

**Example 2**

**Down-regulation of osteopontin and bone sialoprotein II is related to reduced colony formation and metastasis formation of MDA-MB-231 human breast cancer cells**

The expression of OPN and BSP II in a panel of eleven cell lines is shown in Figure 1. The antibody directed against OPN recognized multiple bands with an apparent molecular weight ranging between 60 and 100 kD. It seems noteworthy, that all cell lines expressed OPN, but the expression level varied between low (MDA-MB-231F, BV-173), intermediate (MCF-7, SAOS-2) and high (MDA-MB-231, MDA-MB-435s, T47D, HT-29, HL-60, and ACHN). In addition, the OPN band of K-562 cells varied from all other cell lines in size.

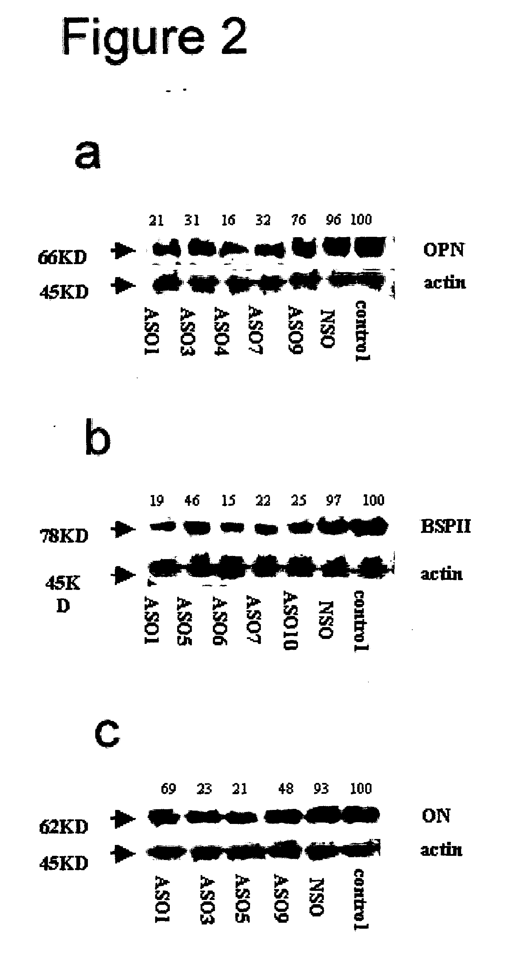

The BSP II antibody recognized two main bands with an apparent molecular weight of 80 kD or higher. Grouping by expression level allowed differentiation between low (MDA-MB-231F), intermediate (K-562), and high levels (all other cell lines). The ON antibody used at first recognized a protein of about 33 kD size. A subsequent antibody, used for experiments as shown in Fig 2C, recognized a protein with a size of about 62 kD. Since the expression of this protein was modulated by ASO exposure (see below) we assume that we observed a dimer.

The Western blot results of protein expression after exposing mammary carcinoma cells to antisense oligonucleotides directed against OPN, BSPII, and ON-mRNA are shown in Table 2 and Figure 2.

## Table 2
### Inhibition of osteopontin, bone sialoprotein II, and osteonectin expression in mammary carcinoma cells following exposure to a series of antisense oligonucleotides (ASOs)

| Target protein | Osteopontin | | Bone sialoprotein II | | | | | Osteonectin | | |
|---|---|---|---|---|---|---|---|---|---|---|
| Cell line | MDA-MB231 | | MDA-MB231 | | MDA-MB231[F] | MDA-MB435s | MDA-MB231 | MDA-MB231 | MDA-MB231[F] | MDA-MB435s |
| Type of exposure | Single [L] | Twofold [L] | Single [L] | Single [E] | Single [L] | Single [L] | Twofold [L] | Single [L] | Single [L] | Single [L] |
| Number of experiments | 2-3 | 2-3 | 1 | 1 | 1 | 1 | 1 | 2-3 | 1 | 1 |
| ASO-1[1] | 25[2)3)] | 34[2)3)] | 45 | 78 | 58 | 16 | 19[2)] | 39[3)] | 44 | 46 |
| ASO-2 | 45 | 60 | 38 | 94 | 94 | 26 | – | 61 | 17 | 64 |
| ASO-3 | 32 | 38 | 97 | 44 | 93 | 22 | – | 25 | 22 | 29 |
| ASO-4 | 16 | 23 | 73 | 55 | 109 | 26 | – | 50 | 31 | 62 |
| ASO-5 | 43 | 80 | 27 | 63 | 101 | 41 | 46 | 30 | 22 | 27 |
| ASO-6 | 45 | 68 | 33 | 16 | 79 | 0 | 15 | 52 | 44 | 56 |
| ASO-7 | 31 | 69 | 58 | 25 | 37 | 51 | 22 | 50 | 32 | 64 |
| ASO-8 | 48 | 97 | 62 | 34 | 101 | 32 | 25 | 54 | 38 | 46 |
| ASO-9 | – | 74 | 35 | 46 | 84 | 49 | – | 37 | 21 | 49 |
| ASO-10 | – | 86 | 33 | 40 | 30 | 39 | – | 46 | 35 | 46 |
| NSO | 99.5 | 99 | 100 | 99 | 100 | 65 | 97 | 94 | 80 | 97 |
| Control | 100 | 100 | 100 | 100 | 100 | 100 | 100 | 100 | 100 | 100 |

[1] The number refers to the corresponding number in table 1 , ASO refers to an antisense oligonucleotide directed against osteopontin, bone sialoprotein II, and osteonectin. For concentration used see Example 1.

[2] X-ray films documenting chemoluminescence bands were scanned, or digitized bands of the respective protein were expressed in relation to untreated control (100%).

[3] mean value

23

MDA-MB231 cells growing without FBS
F) Transfection with lipofectamine
L) Transfection with electroporation
E)

Systematic comparisons showed that ASO-OPN-04, ASO-BSPII-06, and ASO-ON-03 and 05 were the most effective structures within the respective series. Single exposure of cells was enough to reduce the osteopontin expression by 84% in response to ASO-OPN-04. This effect was not increased by repeating the ASO exposure but the protein concentration was kept at a comparable level (77%). A similar efficacy was obtained for ASO-BSPII-06, which caused a by 84% reduced bone sialoprotein expression following single exposure and a by 81% reduced protein level following twofold exposure. Interestingly, MDA-MB-231 cells growing without FBS were less sensitive to ASO exposure, but MDA-MB-435s cells were at least as sensitive as MDA-MB-231 cells.

ASO-ON-03 and 05 were similarly active in reducing osteonectin in MDA-MB-231 cells as shown by decreases of 75% and 70%, respectively. Again, MDA-MB-435s cells were as sensitive as MDA-MB231 cells, but MDA-MB231 cells growing without FBS were more sensitive to ASO exposure.

The inhibition of MDA-MB-231 colony formation is shown in Tables 3-6. Single exposure to the ASOs considered optimal is shown in Table 3.

Table 3

| Inhibition of colony formation of MDA-MB-231 breast cancer cells following single treatment with antisense oligonucleotides directed against osteopontin, bone sialoprotein, osteonectin, and the alkylphosphocholine ErPC$_3$. | | | | | |
|---|---|---|---|---|---|
| **Experiment I** | **Treatment** | **Concentration**[1] | **Mean colony no.** | **SD** | **% Control** |
| | Control | - | 778 [5] | 12 | 100 |
| | ASO-OPN-4 [2] | 5µM | 658 [6] | 48 | 85 |
| | | 10µM | 475 [7] | 12 | 61 |

[1] concentration in medium during exposure of cells; for details see Example 1.

[2] See table 1 for identification

[5] P=0.05 according to the Kruskal-Wallis test versus all treated groups, except ON, 5 µM

[6] P=0.05 according to the Kruskal-Wallis test versus 10 and 20 µM

[7] P=0.0$_5$ according to the Kruskal-Wallis test versus 20 µM

Table 3   (continued)

| Inhibition of colony formation of MDA-MB-231 breast cancer cells following single treatment with antisense oligonucleotides directed against osteopontin, bone sialoprotein, osteonectin, and the alkylphosphocholine ErPC$_3$. | | | | | |
|---|---|---|---|---|---|
| **Experiment I** | **Treatment** | **Concentration**[1] | **Mean colony no.** | **SD** | **% Control** |
| | | 20µM | 416 | 32 | 53 |
| | ASO-BSPII-6 [2] | 5µM | 692 [6] | 12 | 89 |
| | | 10µM | 632 [7] | 19 | 81 |
| | | 20µM | 478 | 55 | 61 |
| | ErPC$_3$ [3] | 10µM | 691 [8] | 9 | 89 |
| | | 14µM | 425 [7] | 28 | 55 |
| | | 20µM | 169 | 16 | 22 |
| **Experiment II** | Control | - | 705 [9] | 14 | 100 |
| | NSO [4] | 20µM | 676 | 15 | 96 |
| | ASO-ON-3 [2] | 5µM | 648 [6] | 42 | 92 |
| | | 10µM | 576 [7] | 28 | 82 |
| | | 20µM | 514 | 43 | 73 |
| | ErPC$_3$ | 10µM | 621 [8] | 10 | 88 |
| | | 14µM | 385 [7] | 24 | 55 |
| | | 20µM | 173 | 17 | 25 |

[1] concentration in medium during exposure of cells; for details see Example 1.

[2] See table 1 for identification

[3] Erucylphospho-NNN-trimethylpropanolamine

[4] nonsense oligonucleotide

[6] P=0.05 according to the Kruskal-Wallis test versus 10 and 20 µM

[7] P=0.0$_5$ according to the Kruskal-Wallis test versus 20 µM

[8] P=0.05 according to the Kruskal-Wallis test versus 14 and 20 µM

[9] P=0.05 according to the Kruskal-Wallis test versus all treated groups of experiment II, except NSO

In contrast to ErPC$_3$ which was used as a standard, effecting 11% (lowest concentration) to 78% (highest concentration, P=0.05, respectively) colony growth inhibition, the ASOs were less effective. Nevertheless, all ASOs reduced the colony growth concentration dependently, and this effect was significantly different from untreated control (P=0.05) except for ASO-ON-03 at 5 µM. ASO-OPN-04 caused 47% colony growth inhibition at the maximum concentration of 20 µM, ASO-BSPII-6 (39%), and ASO-ON-3 (27%) were even less effective at equimolar highest concentrations (20µM). Serial combined exposure (Table 4) to ASO-OPN-04 (first agent) and ErPC$_3$ (second agent) resulted in colony growth inhibition compared to untreated control ranging from 33% (5 µM ASO OPN-4 + 10 µM ErPC$_3$) to 93% (20 µM ASO-OPN-4 + 20µM ErPC$_3$). Similarly, combination of ASO-BSPII-06 with ErPC$_3$ caused reduction in clonogenic growth ranging from 28% (5 µM ASO-BSPII-06 + 10 µM ErPC$_3$) to 87% (20 µM ASO-BSPII-06 + 20 µM ErPC$_3$). Finally, combination of the ASO-ON-03 with ErPC$_3$ caused colony growth inhibition ranging from 14% (5 µM ASO-ON-03 + 10 µM ErPC$_3$) to 84% (20 µM ASO-ON-03 + 20 µM ErPC$_3$).

Table 4

| Inhibition of colony formation of MDA-MB-231 breast cancer cells after exposure to antisense oligonucleotides directed against OPN, BSPII and ON followed exposure to the alkylphosphocholine $ErPC_3$. | | | | | |
|---|---|---|---|---|---|
| **Treatment 1** | **Treatment 2** | **Concentration [1] ASO + $ErPC_3$** | **Mean colony no.** | **SD** | **% Control** |
| Control | | - | 778 [4] | 12 | 100 |
| ASO-OPN-4 [2] | $ErPC_3$ [3] | 5µM + 10µM | 518 [5] | 21 | 67 |
| | | 10µM+10µM | 451 [6] | 22 | 58 |
| | | 20µM+10µM | 382 | 31 | 49 |
| | | 5µM + 14µM | 393 [6] | 8 | 51 |
| | | 10µM+14µM | 362 [6] | 39 | 47 |
| | | 20µM+14µM | 221 | 15 | 28 |
| | | 5µM + 20µM | 150 [5] | 11 | 19 |
| | | 10µM+20µM | 118 [6] | 11 | 15 |
| | | 20µM+20µM | 56 | 17 | 7 |
| ASO-BSPII-6 [2] | | 5µM + 10µM | 563 [5] | 23 | 72 |
| | | 10µM+10µM | 497 [6] | 20 | 64 |
| | | 20µM+10µM | 404 | 4 | 52 |
| | | 5µM + 14µM | 393 [6] | 13 | 51 |
| | | 10µM+14µM | 374 | 50 | 48 |
| | | 20µM+14µM | 315 | 40 | 40 |
| | | 5µM + 20µM | 168 [5] | 27 | 22 |
| | | 10µM+20µM | 131 [6] | 14 | 17 |
| | | 20µM+20µM | 102 | 12 | 13 |
| Control | | | 705 [4] | 13 | 100 |
| ASO-ON-3 [2] | $ErPC_3$ | 5µM + 10µM | 606 [5] | 10 | 86 |
| | | 10µM+10µM | 511 | 38 | 54 |
| | | 20µM+10µM | 489 | 4 | 24 |
| | | 5µM + 14µM | 379 | 16 | 75 |
| | | 10µM+14µM | 336 | 32 | 48 |
| | | 20µM+14µM | 375 | 27 | 21 |
| | | 5µM + 20µM | 168 [6] | 26 | 70 |
| | | 10µM+20µM | 141 | 28 | 52 |

[1] concentration in medium during exposure of cells; for details see Example 1.

[2] See table 1 for identification

[3] Erucylphospho-NNN-trimethylpropanolamine

[4] P=0.05 according to the Kruskal-Wallis test versus all treated groups

[5] P=0.05 according to the Kruskal-Wallis test versus 10 and 20 µM ASO

[6] P=0.05 according to the Kruskal-Wallis test versus 20 µM ASO

Table 4   (continued)

| Inhibition of colony formation of MDA-MB-231 breast cancer cells after exposure to antisense oligonucleotides directed against OPN, BSPII and ON followed exposure to the alkylphosphocholine ErPC$_3$. | | | | | |
|---|---|---|---|---|---|
| Treatment 1 | Treatment 2 | Concentration [1] ASO + ErPC$_3$ | Mean colony no. | SD | % Control |
| | | 20µM+20µM | 113 | 20 | 16 |

[1] concentration in medium during exposure of cells; for details see Example 1.

Repeated exposure to ASO-OPN-04 (Tab. 5) was more effective than single exposure to this agent, as shown by colony number reduction. Twofold exposure to ASO-OPN-04 at 5 µM reduced colony counts by 16%, and by 60% at 20 µM. Interestingly, repeated exposure to ASO-BSP II-06 caused no increased inhibition of colony formation as compared with single exposure. The combination of this treatment schedule plus a final exposure to ErPC$_3$ (14 µM) caused an additive effect, as shown by 76% to 83% (ASO-OPN-04) and 61% to 75% (ASO-BSPII-06) reduced colony numbers, respectively (Table 5).

Table 5

| Inhibition of colony formation of MDA-MB-231 breast cancer cells after combination treatment with repeated exposure to antisense oligonucleotides directed against osteopontin or bone sialoprotein, followed by the alkylphosphocholine ErPC$_3$. | | | | | |
|---|---|---|---|---|---|
| Experiment III a | Treatment (abbreviation) | Concentration [1] | Mean colony no. | SD | % Control |
| | Control | - | 456[4] | 6 | 100 |
| | NSO | 20µM | 430 | 20 | 94 |
| | Lipofectamine[2] | | 437 | 26 | 96 |
| | ErPC3 | 14µM | 239[5] | 16 | 52 |
| | ASO-OPN-4 [3] | 2 x 5µM | 381[6,8] | 33 | 84 |
| | | 2 x 10µM | 233[7,8] | 25 | 51 |
| | | 2 x 20µM | 181[7] | 18 | 40 |
| | ASO-BSPII-6 [3] | 2 x 5µM | 415[6,8] | 11 | 91 |
| | | 2 x 10µM | 373[8] | 25 | 82 |
| | | 2 x 20µM | 326[8] | 28 | 72 |
| | 2x ASO-OPN-4 + ErPC$_3$ | 5µM + 14µM | 110 [9] | 11 | 24 |
| | 2x ASO-OPN-4 + ErPC$_3$ | 10µM + 14µM | 90 [9] | 10 | 20 |
| | 2x ASO-OPN-4 + ErPC$_3$ | 20µM + 14µM | 71 [4] | 8 | 17 |

[1] concentration in medium during exposure of cells; for details see Example 1.

[2] control with lipofectamine

[3] See table 1 for identification

[4] P=0.05 versus all groups except NSO and lipofectamine.

[5] P=0.05 versus all combinations with ErPC$_3$.

[6] P=0.05 versus ErPC$_3$ (2x 10; 2 x20 µM)

[7] P=0.05 versus ErPC$_3$ (2x 20 µM)

[8] P=0.05 versus the respective combination with ErPC$_3$.

[9] P=0.05 versus 2x OPN (20 µM) + ErPC$_3$ (14 µM).

Table 5   (continued)

| Experiment III a | Treatment (abbreviation) | Concentration [1] | Mean colony no. | SD | % Control |
|---|---|---|---|---|---|
| | 2x ASO-BSPII-6 + ErPC$_3$ | 5µM + 14µM | 180[10] | 16 | 39 |
| | 2x ASO-BSPII-6 + ErPC$_3$ | 10µM + 14µM | 136 [4] | 8 | 30 |
| | 2x ASO-BSPII-6 + ErPC$_3$ | 20µM + 14µM | 114[4] | 17 | 25 |

*Inhibition of colony formation of MDA-MB-231 breast cancer cells after combination treatment with repeated exposure to antisense oligonucleotides directed against osteopontin or bone sialoprotein, followed by the alkylphosphocholine ErPC$_3$.*

[1] concentration in medium during exposure of cells; for details see Example 1.

[4] P=0.05 versus all groups except NSO and lipofectamine.

[10] P=0.05 versus 2x BSP II (10 µM) + ErPC$_3$ (14 µM) and 2x BSP II (20 µM) + ErPC$_3$ (14 µM).

In a subsequent experiment the sequence of agents was reversed (Table 6). Within normal experimental variation, ErPC$_3$ followed by twofold exposure to ASO-OPN-04 or ASO-BSPII-06 was as effective as the reverse sequence in the preceding experiment and all combinations caused significant colony growth inhibition as compared with the respective control.

Table 6

| Experiment III b | Treatment | Concentration[1] | Mean colony no. | SD | % Control |
|---|---|---|---|---|---|
| | Control | - | 517[4] | 32 | 100 |
| | NSO | 20µM | 504 | 12 | 97 |
| | Lipofectamine[2] | | 505 | 38 | 97 |
| | ErPC$_3$ | 14µM | 311[5] | 20 | 59 |
| | ASO-OPN-4 [3] | 2x 5µM | 330[6,8] | 14 | 63 |
| | | 2x 10µM | 296[7,8] | 12 | 57 |
| | | 2x 20µM | 258[8] | 5 | 50 |
| | ASO-BSPII-6 [3] | 2x 5µM | 363[6,8] | 8 | 70 |
| | | 2x 10µM | 282[8] | 14 | 55 |
| | | 2x 20µM | 251[8] | 18 | 49 |
| | ErPC$_3$ + 2x ASO-OPN | 14µM + 5µM | 177[9] | 16 | 34 |
| | ErPC$_3$ + 2x ASO-OPN | 14µM + | 154 | 12 | 30 |

*Inhibition of colony formation of MDA-MB-231 breast cancer cells after treatment with the alkylphosphocholine ErPC$_3$, followed by combination treatment with repeated exposure to antisense oligonucleotides directed against osteopontin or bone sialoprotein*

[1] concentration in medium during exposure of cells; for details see Example 1.

[2] control with lipofectamine

[3] µSee table 1 for identification

[4] P=0.05 versus all groups except NSO and lipofectamine.

[5] P=0.05 versus all combinations with ErPC$_3$

[6] P=0.05 versus 2x 10 and 2 x20 µM.

[7] P=0.05 versus 2x 20 µM.

[8] P=0.05 versus all combinations with ErPC$_3$

[9] P=0.05 versus 2x OPN (20 µM) + ErPC$_3$ (14 µM).

Table 6   (continued)

| Inhibition of colony formation of MDA-MB-231 breast cancer cells after treatment with the alkylphosphocholine ErPC$_3$, followed by combination treatment with repeated exposure to antisense oligonucleotides directed against osteopontin or bone sialoprotein | | | | | |
|---|---|---|---|---|---|
| Experiment III b | Treatment | Concentration[1] | Mean colony no. | SD | % Control |
| | ErPC$_3$ + 2x ASO-OPN | 10μM 14μM + 20μM | 137 | 11 | 26 |
| | ErPC$_3$ + 2x ASO-BSPII | 14μM + 5μM | 188 [10] | 17 | 36 |
| | ErPC$_3$ + 2x ASO-BSPII | 14μM + 10μM | 165 [10] | 8 | 32 |
| | ErPC$_3$ + 2x ASO- BSPII | 14μM + 20μM | 138 | 14 | 27 |

[1] concentration in medium during exposure of cells; for details see Example 1.

[10] P=0.05 versus 2x BSP II (10 μM) + ErPC$_3$ (14 μM).

Isobologram analysis of all combinations yielded values not significantly different from 1, thus indicating linear additivity of the combination effects.

The results of the bioassay are shown in Table 7. Exposure to the ASO-OPN-04 and ASO-BSPII-06 for three days before implantation into nude rats caused a significantly reduced tumor take rate, as assessed by the appearance of osteolytic lesions following exposure to ASO-OPN-04, and a reduced size of osteolytic lesions in the X-ray positive rats following both agents.

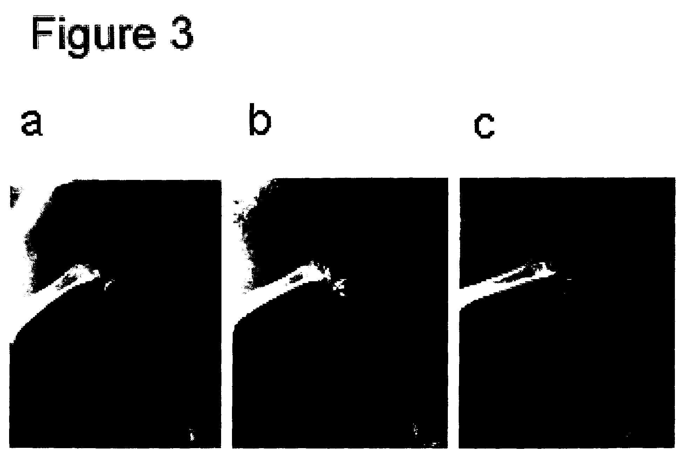

Nude rats that received $1 \times 10^5$ untreated or NSO-treated MDA-MB-231 breast cancer cells developed bone metastasis in four of four cases (Table 7; c.f. Fig. 3). The metastatic lesions were detectable for the first time after four weeks [mean size of the two control groups: 197 relative units (R.U)] and doubled in size until week eight (mean size of the two control groups: 416 R.U.). Three of four rats that received $1 \times 10^5$ MDA-MB-231 breast cancer cells pretreated with the ASO directed against OPN, developed no discernible metastasis within the observation period (p=0.028). One of the four rats developed lytic metastasis, but this lesion was distinctly smaller in size than those in control rats (week 4: 34 R.U., 17% of control; week 8: 60 R.U., 17% of control; p=0.05). Similarly, two of four rats that received $1 \times 10^5$ MDA-MB-231 breast cancer cells pretreated with the ASO directed against BSP II remained free of visible metastasis within the observation period of eight weeks, one rat showed lytic metastasis after four weeks already (14 R.U.,7% of control ), an other rat developed a metastasis after eight weeks (63 R.U (mean), 15% of control; p=0.05). Thus, exposure to both, ASO-OPN-04 and ASO-BSPII-06, was associated with a significantly reduced lesion size, and the former agent caused a significantly reduced incidence of lesions, as well.

**Table 7**
**Overview of bioassay results**

| Group No. | Treatment [1] | Animal No. | Observation period (weeks) | Incidence of metastasis | Size of lesion [2] | Mean | T/Cx100 [3] |
|---|---|---|---|---|---|---|---|
| 1 | ASO-OPN-4 | 4 | 4 | 1/4 | 0, 0, 0, 34 | 8.5 (±17) [6] | 3.9 |
| | | | 6 | 1/4 | 0, 0, 0, 46 | | 3.8 |
| | | | 8 | 1/4[4] | 0, 0, 0, 60 [5] | 11.5 (±23) | 4.3 |
| | | | | | | 15 (±30) | |
| 2 | ASO-BSP II-6 | 4 | 4 | 1/4 | 0, 0, 0, 14 | 3.5 (±7) | 1.6 |
| | | | 6 | 1/4 | 0, 0, 0, 35 | 8.8 (±18) | 2.9 |
| | | | 8 | 2/4 | 0, 0, 20, 106[5] | 31.5 (±51) | 9.1 |
| 3 | NSO | 2 | 4 | 2/2 | 106, 240 | 173 (134) [7] | 78 |
| | | | 6 | 2/2 | 162, 411 | | 95 |
| | | | 8 | 2/2 | 242,728 | 287 (249) | 140 |
| | | | | | | 485 (486) | |
| 4 | Control | 2 | 4 | 2/2 | 314, 127 | 221 (187) | 100 |
| | | | 6 | 2/2 | 386, 218 | 302 (168) | 100 |
| | | | 8 | 2/2 | 425, 266 | 346 (159) | 100 |

[1] GFP-MDA-MB-231 cells were exposed to ASOs or NSO (for details see ) before transplantation to nude rats

[2] Product of pixel number and mean black intensity  in individual rats as determined by image analysis from the X-ray radiograph.

[3] Mean lesion size of treated over control rats times 100

[4] P=0,0028 versus control groups 3 and 4 ($\chi^2$ -test)

[5] P=0.05 versus control groups 3 and 4 (Kruskal-Wallis test).

18

<sup>6)</sup> Numbers in brackets denote standard deviation
<sup>7)</sup> Numbers in brackets denote range

Conclusions

For OPN, the ASOs directed against the start codon (ASO-OPN-01) and a loop after the stop codon (ASO-OPN-04) were most effective in down regulating the protein. The fact that an ASO directed towards a sequence after the stop codon is active in suppressing the expression of OPN hints to an activity against pre-mRNA or a triplex formation with DNA and that sufficient concentrations are obtained in the nucleus. The comparison of two overlapping ASOs directed against four target structures, respectively, shows no significant differences between the almost identical ASOs, except for ASO-OPN-04 and ASO-OPN-05. The superiority of ASO-OPN-04 over ASO-OPN-05 might be explained by the increased accessibility of the respective target structures: whereas ASO-OPN-04 can hybridize with 17 nucleotides (85% of total ASO length) to an accessible target site, ASO-OPN-05 can do so only with 14 nucleotides (70% of total ASO-length).

For BSP II, the ASO-pair directed against the region of the start codon (ASO-BSP II-01 and ASO-BSP II-02) was less effective than those directed against the largest loop of the molecule (ASO-BSP II-05 and ASO-BSP II-06). From these two, ASO-BSP II-06 was more effective, in line with its hybridization to a closely neighboring loop. Also, the ASO pair directed to the region of the stop codon was more effective than the average with ASO-10 being slightly superior to ASO-BSPII-09, presumably because ASO-10 can hybridize to the loop as well as a pair of unpaired nucleotides.

For ON, the ASO directed against the start codon (ASO-ON-1) was of average activity, as well as those directed to sites after the stop codon (ASO-ON-08 - ASO-ON-10). The highest activity was associated with two ASOs (ASO-ON-03 and ASO-ON-05) spanning over a small series of bulges. However, no ASO was really outstanding over all other structures.

With regard to the transfection method used, differences resulting in the resulting protein level were observed in this study, e.g. for ASO-BSP II-05 and -07 when using lipofectamine and electrotransfection (Table 2). Overall, however, the effects were comparable, although the effective ASO concentrations were at least tenfold lower when using lipofectamine.

When comparing the most effective ASOs, as assessed from Western blot, for their activity to reduce colony formation of MDA-MB-231 cells, the efficacy decreased at equimolar levels from OPN (ASO-OPN-04) to BSP II (ASO-BSP II-06) and ON (ASO-ON-03). This difference could result either from differential suppression of the respective protein levels (ASO-OPN-04: 85%; ASO-BSP 11-06: 75%; ASO-ON-03: 75%) or from a differential importance of the target proteins supporting colony growth. Colony formation reflects the ability of a specialized compartment of tumor cells to grow anchorage independently and to reproduce themselves in the absence of neighboring cells. Therefore, inhibition of colony formation is indicative of an inhibition of autocrine stimulation. It is noteworthy, that reduced levels of all three proteins investigated were associated with inhibition of colony formation, indicating that OPN, BSPII, and ON acted as direct or indirect mitogens. Since the treatment effect of the ASOs are clearly reversible, as shown by increased protein expression levels after five days and gain of migrating functions, the effects seen after 5-7 days (clonogenicity) and 28 days (in vivo) are related to an early inefficiency of the cells to form colonies, to migrate, or to seed into the bone compartment. The extent of response to the ASOs, which was additively increased by repeated exposure, was clearly dependent on the respective concentrations used. In combination with ErPC3 all ASOs showed additive activity, as can be derived from their combination index, respectively, that varied around 1. This is in line with the assumption that both combination partners act on different targets.

Furthermore, the result show that exposure to an ASO directed against OPN was instrumental in inhibiting the formation of osteolytic lesions in three of four animals used, and in reducing the lesion size of the remaining rat. A similar effect in response to down modulating BSP II-levels has not been described so far. Although slightly less active, the reduction of BSP II levels caused an effect which parallels that of reducing OPN levels. These results suggest that reduction of OPN and BSP II levels in vivo could be instrumental for a combination treatment of cancer, specifically of osteolytic metastasis. In conclusion, suited ASOs reduced the respective protein levels of OPN, BSPII, and ON specifically. Their activities in inhibiting colony growth and formation of osteolytic bone metastasis suggest a beneficial effect in restraining the growth of metastasis in vivo, as well.

**Literature**

1. Agrawal D, Chen T, Irby R, Quackenbush J, Chambers AF, Szabo M, Cantor A, Coppola D, Yeatman TJ.
Osteopontin identified as lead marker of colon cancer progression, using pooled sample expression profiling. J Natl Cancer Inst 2002;94:513-21

2. Rudland PS, Platt-Higgins A, El-Tanani M, De Silva Rudland S, Barraclough R, Winstanley JH, Howitt R, West CR.
Prognostic significance of the metastasis-associated protein osteopontin in human breast cancer. Cancer Res 2002;62:3417-27

3. Tuck AB, Chambers AF
The role of osteopontin in breast cancer: clinical and experimental studies.
J Mammary Gland Biol Neoplasia 2001; 6:419-27.

4. Fisher LW, Torchia DA.
Flexible structures of SIBLING proteins, bone sialoprotein, and osteopontin.
Biochem Biophys Res Commun 2001;280:460-65

5. Diel IJ, Solomayer EF, Seibel MJ, Pfeilschifter J, Maisenbacher H, Gollan C, Pecherstorfer M, Conradi R, Kehr G, Boehm E, Armbruster FP, Bastert G.
Serum bone sialoprotein in patients with primary breast cancer is a prognostic marker for subsequent bone metastasis. Clin Cancer Res 1999 Dec;5:3914-19

6. Sharp JA, Sung V, Slavin J, Thompson EW, Henderson MA.
Tumor cells are the source of osteopontin and bone sialoprotein expression in human breast cancer. Lab Invest 1999 79:869-77

7. Waltregny D, Bellahcene A, Van Riet I, Fisher LW, Young M, Fernandez P, Dewe W, de Leval J, Castronovo V.
Prognostic value of bone sialoprotein expression in clinically localized human prostate cancer. J Natl Cancer Inst 1998;90:1000-8

8. Fedarko NS, Jain A, Karadag A, Van Eman MR, Fisher LW.
Elevated serum bone sialoprotein and osteopontin in colon, breast, prostate, and lung cancer. Clin Cancer Res 2001;7:4060-66

9. Rosol TJ.
Pathogenesis of bone metastases: role of tumor-related proteins.
J Bone Miner Res 2000;15:844-50

10. Porte H, Chastre E, Prevot S, Nordlinger B, Empereur S, Basset P, Chambon P, Gespach C.
Neoplastic progression of human colorectal cancer is associated with overexpression of the stromelysin-3 and BM-40/SPARC genes. Int J Cancer 1995;64:70-5

11. Yamanaka M, Kanda K, Li NC, Fukumori T, Oka N, Kanayama HO, Kagawa S.
Analysis of the gene expression of SPARC and its prognostic value for bladder cancer. J Urol 2001;166:2495-9

12. Jacob K, Webber M, Benayahu D, Kleinman HK.
Osteonectin promotes prostate cancer cell migration and invasion: a possible mechanism for metastasis to bone.
Cancer Res 1999;59:4453-7

13. Zuker M.
On finding all suboptimal foldings of an RNA molecule. Science 1989;244:48-52

14. Devereux J, Haeberli P, Smithies O.
A comprehensive set of sequence analysis programs for the VAX.
Nucleic Acids Res 1984;12:387-95

15. Senger M, Glatting KH, Ritter O, Suhai S.
X-HUSAR, an X-based graphical interface for the analysis of genomic sequences.
Comput Methods Programs Biomed 1995;46:131-41

16. Dunn, O.J.
Multiple comparison using rank sums. Technometrics 1964; 6: 241-252,

17. Berenbaum MC.
What is synergy? Pharmacol Rev 1989; 41:93-141

18.Chou TC, Talalay P.
Quantitative analysis of dose-effect relationships: the combined effects of multiple drugs or enzyme inhibitors.
Adv Enzyme Regul 1984;22:27-55

**Claims**

1. A pharmaceutical composition containing at least one compound 8 to 50 nucleobases in length targeted to (a) nucleic acid molecule(s) encoding osteopontin, bone sialoprotein II and/or osteonectin, wherein said compound (s) specifically hybridize(s) with and inhibit(s) the expression of the nucleic acid(s) encoding osteopontin, bone sialoprotein II and/or osteonectin.

2. The pharmaceutical composition of claim 1, wherein said compound is (a) an antisense oligonucleotide or (b) a vector allowing to transcribe an antisense oligonucleotide of (a).

**3.** The pharmaceutical composition of claim 1 or 2, wherein said compound has the nucleic acid sequence (a) 5'-CTA ACT TAA AAA ACA AAA GA-3' (ASO-OPN-04), 5'-GCT-TTC-TTC-GTT-TTC-ATT-TC-3' (ASO-BSP 11-06), 5'-GGG GGC TGG GCA GCT GGT GG-3' (ASO-ON-03) or 5'-ATG TTA TAG TTC TTC TCG AA-3' (ASO-ON-05) or (b) a nucleic acid sequence which has at least 70% identity with a nucleic acid sequence of (a).

**4.** The pharmaceutical composition of any one of claims 1 to 3, wherein said compound has a length of 15 to 25 nucleobases.

**5.** The pharmaceutical composition of any one of claims 1 to 4 wherein said antisense oligonucleotide comprises at least one modified internucleoside linkage.

**6.** The pharmaceutical composition of claim 5 wherein said internucleoside linkage is a phosphorothioate linkage.

**7.** The pharmaceutical composition of any one of claims 1 to 6 wherein said antisense oligonucleotide comprises at least one modified sugar moiety.

**8.** The pharmaceutical composition of claim 7 wherein said modified sugar moiety is a 2'-O-methoxyethyl sugar moiety.

**9.** The pharmaceutical composition of any one of claims 2 to 8 wherein said antisense oligonucleotide comprises at least one modified nucleobase.

**10.** The pharmaceutical composition of claim 9 wherein said modified nucleobase is a 5-methylcytosine.

**11.** The pharmaceutical composition of any one of claims 1 to 10 furthermore containing a cytostatic alkylphosphocholine.

**12.** The pharmaceutical composition of claim 11 wherein said cytostatic alkylphosphocholine is $ErPC_3$.

**13.** The pharmaceutical composition of any one of claims 2 to 11 wherein said antisense oligonucleotide is encapsulated into a liposome.

**14.** Use of the compound(s) as defined in any one of claims 1 to 13 for the preparation of a pharmaceutical composition for the treatment or prevention of bone cancer or metastasis of several cancer types.

**15.** Use according to claim 14 wherein said cancer types are cancer of the breast, colon, prostate, bone, urinary bladder or colorectal cancer.

**16.** Use according to claim 14 or 15 wherein said cancer metastasis is a lytic bone metastasis.

# Figure 1

## a

OPN

## b

BSP II

# Figure 2

## a

## b

## c

# Figure 3

a        b        c

## European Patent Office · EUROPEAN SEARCH REPORT

Application Number

EP 03 02 0789

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 98 29138 A (ADRIS SORAYA KARINA ;BRAVO ALICIA INES (AR); MORDOH JOSE (AR); INS) 9 July 1998 (1998-07-09) | 1-10, 13-16 | C12N15/11 C07H21/00 A61K31/711 A61P35/00 A61P35/04 |
| Y | *claims* * page 4, paragraph 4 - page 7, paragraph 4 * | 11,12 | |
| X | WO 02 25285 A (SMITH ARTHUR JOHN ;UNIV LIVERPOOL (GB); RUDLAND PHILIP SPENCER (GB) 28 March 2002 (2002-03-28) | 1-10, 13-16 | |
| Y | * page 2, paragraph 2 - page 3, line 1 * *claims* * page 7, paragraph 3 - page 8, paragraph 1 * | 11,12 | |
| X | WO 02 100899 A (ARMBRUSTER FRANZ PAUL ;PAULSSON MATS (DE); BERGER MARTIN R (DE); F) 19 December 2002 (2002-12-19) | 1-10, 13-16 | |
| Y | *claims* *examples* | 11,12 | TECHNICAL FIELDS SEARCHED (Int.Cl.7) |
| X | ADWAN HASSAN ET AL: "Inhibition of cell migration following exposure to antisense oligonucleotides against osteopontin, bone sialoprotein, and osteonectin." PROCEEDINGS OF THE AMERICAN ASSOCIATION FOR CANCER RESEARCH ANNUAL, vol. 44, July 2003 (2003-07), page 56 XP001179152 94th Annual Meeting of the American Association for Cancer Research;Washington, DC, USA; July 11-14, 2003, July 2003 ISSN: 0197-016X * abstract * | 1-10, 13-16 | C12N C07H A61K A61P |

-/--

The present search report has been drawn up for all claims

| Place of search | Date of completion of the search | Examiner |
|---|---|---|
| MUNICH | 16 February 2004 | Böhmerova, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

## EUROPEAN SEARCH REPORT

**European Patent Office**

**Application Number**

EP 03 02 0789

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WEBER G F: "The metastasis gene osteopontin: A candidate target for cancer therapy" BIOCHIMICA ET BIOPHYSICA ACTA - REVIEWS ON CANCER 28 DEC 2001 NETHERLANDS, vol. 1552, no. 2, 28 December 2001 (2001-12-28), pages 61-85, XP002270300 ISSN: 0304-419X * abstract * * page 69, left-hand column, paragraph 2 - page 70, left-hand column, paragraph 1 * --- | 1-10, 13-16 | |
| Y | JENDROSSEK VERENA ET AL: "Structure-activity relationships of alkylphosphocholine derivatives: Antineoplastic action on brain tumor cell lines in vitro" CANCER CHEMOTHERAPY AND PHARMACOLOGY, vol. 50, no. 1, July 2002 (2002-07), pages 71-79, XP002270301 ISSN: 0344-5704 * the whole document * --- | 11,12 | |
| Y | KONSTANTINOV SPIRO M ET AL: "Combination with an antisense oligonucleotide synergistically improves the antileukemic efficacy of erucylphospho-N,N,N-trimethylpropylammonium in chronic myeloid leukemia cell lines" MOLECULAR CANCER THERAPEUTICS, vol. 1, no. 10, August 2002 (2002-08), pages 877-884, XP002270302 ISSN: 1535-7163 * abstract; figures 3,4 * --- -/-- | 11,12 | TECHNICAL FIELDS SEARCHED (Int.Cl.7) |

The present search report has been drawn up for all claims

| Place of search | Date of completion of the search | Examiner |
|---|---|---|
| MUNICH | 16 February 2004 | Böhmerova, E |

EPO FORM 1503 03.82 (P04C01)

## EUROPEAN SEARCH REPORT

European Patent Office

**Application Number**

EP 03 02 0789

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | PRITCHARD ET AL: "Is the expression of osteopontin and bone sialoprotein greater in breast cancer bone metastases compared to other metastatic sites" EUROPEAN SYMPOSIUM ON CALCIFIED TISSUES, vol. 20, no. 4S, 25 April 1997 (1997-04-25), page 63S XP002107804 * abstract * | 1-16 | |

-----

**TECHNICAL FIELDS SEARCHED (Int.Cl.7)**

The present search report has been drawn up for all claims

| Place of search | Date of completion of the search | Examiner |
|---|---|---|
| MUNICH | 16 February 2004 | Böhmerova, E |

EPO FORM 1503 03.82 (P04C01)

## EP 1 514 929 A1

### ANNEX TO THE EUROPEAN SEARCH REPORT
### ON EUROPEAN PATENT APPLICATION NO.

EP 03 02 0789

This annex lists the patent family members relating to the patent documents cited in the above-mentioned European search report.
The members are as contained in the European Patent Office EDP file on
The European Patent Office is in no way liable for these particulars which are merely given for the purpose of information.

16-02-2004

| Patent document cited in search report | | Publication date | Patent family member(s) | | Publication date |
|---|---|---|---|---|---|
| WO 9829138 | A | 09-07-1998 | AU | 5333298 A | 31-07-1998 |
| | | | EP | 0950097 A2 | 20-10-1999 |
| | | | WO | 9829138 A2 | 09-07-1998 |
| WO 0225285 | A | 28-03-2002 | AU | 8429701 A | 02-04-2002 |
| | | | CA | 2422568 A1 | 28-03-2002 |
| | | | EP | 1319186 A1 | 18-06-2003 |
| | | | WO | 0225285 A1 | 28-03-2002 |
| WO 02100899 | A | 19-12-2002 | WO | 02100899 A2 | 19-12-2002 |
| | | | WO | 02100901 A2 | 19-12-2002 |

EPO FORM P0459

For more details about this annex : see Official Journal of the European Patent Office, No. 12/82

29